# EUROPÄISCHE PATENTSCHRIFT

(11) **EP 1 399 096 B1**
(45) Veröffentlichungstag und Bekanntmachung des Hinweises auf die Patenterteilung: **02.07.2008**
(21) Anmeldenummer: 02750898.5
(22) Anmeldetag: 07.05.2002
(51) Int. Cl.: A61F 2/08

(54) **SEHNENERSATZ-IMPLANTAT**
IMPLANT
IMPLANT

(30) Priorität: 29.06.2001 DE 10131699
(43) Veröffentlichungstag der Anmeldung: 24.03.2004
(73) Patentinhaber: Tutogen Medical GmbH, 91077 Neunkirchen am Brand (DE)
(72) Erfinder: LÖWELL, Matthias, 90408 Nürnberg (DE); LANDIS, Klaus, 90562 Heroldsberg (DE); HENNINGER, Jürgen, 63069 Offenbach (DE)
(74) Vertreter: Manitz, Finsterwald & Partner GbR
(86) Internationale Anmeldenummer: PCT/EP2002/005033
(87) Internationale Veröffentlichungsnummer: WO 2003/003942

(56) Entgegenhaltungen:
- EP-A- 1 013 239
- WO-A-01/50999
- WO-A-98/12995
- FR-A- 2 743 294
- US-A- 5 067 962
- US-A- 6 090 998

## Beschreibung

Die vorliegende Erfindung betrifft ein Implantat zum Ersatz von Sehnen, insbesondere des vorderen Kreuzbandes, nach dem Oberbegriff des Anspruchs 1 (vgl. US-A-6,090,998).

Bei teilweise oder vollständiger Ruptur des Kreuzbandes wird üblicherweise als Kreuzbandersatz in das Kniegelenk ein autologer bzw. allogener Patellaknochenblock-Patellasehne-Tibiaknochenblock-Verbund implantiert. Zur Fixation der Knochenblöcke werden in den Femur und die Tibia Bohrungen eingebracht, in die die Knochenblöcke implantiert werden. Die Fixation der implantierten Knochenblöcke in den Bohrkanälen im Femur bzw. in der Tibia erfolgt mit entsprechend dimensionierten Interferenzschrauben, die beispielsweise aus Metall (z.B. Titan) oder aus Kunststoffen (z.B. Polylactid) bestehen können.

Diese Methode hat jedoch Nachteile. So besteht bei der Fixation mit metallischen Interferenzschrauben die Gefahr, daß während der Implantation ein Knochenblock oder ein Sehnenabschnitt des Implantats verletzt bzw. zerstört wird. Außerdem verbleiben die Interferenzschrauben lebenslänglich im Implantatlager und führen bei diagnostischen Untersuchungen, wie z.B. Röntgenuntersuchungen, zu Artefakten in den durch die Untersuchung erhaltenen Bildern. Insbesondere ist daher nach einer Implantation eine Diagnose über den Einheilungserfolg nur bedingt möglich.

Beim Ersatz von Sehnen können zwar prinzipiell auch Implantate oder Teile derselben sowie die Fixationselemente aus Kunststoffen verwendet werden. Diese werden aber über Monate hinweg abgebaut, wobei die dabei entstehenden Mono- bzw. Oligomere aber zu einer Schädigung des Implantats oder des Implantatlagers, d.h. des Knochens, mit entsprechenden Komplikationen, wie z.B. einer Lyse des Knochens, führen können.

Aus der US 5,067,962 ist ein Implantat zum Ersatz von Schnen bekannt, das einen ersten und einen zweiten Grundkörper aus Knochenmaterial aufweist, die über ein Kollagenband verbunden sind. Die Grundkörper sind konisch ausgebildet und werden mittels eines Stahlstiftes im Implantatlager verstiftet.

Aufgabe der vorliegenden Erfindung ist es, ein Implantat zum Ersatz von Sehnen zu schaffen, bei dessen Implantation die Gefahr einer Beschädigung des Implantats bei der Fixation stark reduziert ist und bei dem eine Artefaktbildung bei diagnostischen Verfahren und eine Schädigung des Implantatlagers nach der Implantation weitgehend vermieden werden.

Die Aufgabe wird gelöst durch ein Implantat zum Ersatz von Sehnen mit den Merkmalen nach Anspruch 1.

Das erfindungsgemäße Implantat weist zwei Grundkörper aus Knochenmaterial menschlichen oder tierischen Ursprungs auf, die über ein flexibles Kollagenband verbunden sind. Bei dem Knochenmaterial kann es sich jeweils um spongiöses und/oder kortikales und/oder kortiko-spongiöses Material handeln, wobei die beiden Grundkörper grundsätzlich aus verschiedenen Knochenmaterialien gebildet sein können. Die Grundkörper können auch als Verbund aus verschiedenen der genannten Knochenmaterialien gebildet sein.

Das flexible Kollagenband ist an den Grundkörpern fixiert, wobei die Fixation je nach Wahl des Ausganematerials für das Implantat natürlich, zum Beispiel bei Verwendung eines Knochens mit angewachsener Sehne, oder künstlich sein kann. Bei einer künstlichen Verbindung kann die Verbindung von Grundkörper und Kollagenband beispielsweise mittels biologischer Kleber oder mechanischer Fixationselemente wie Schrauben und Pins erfolgen. Bevorzugt sind jedoch natürliche Knochenverbünde.

Erfindungsgemäß sind die beiden Grundkörper zur Fixation in entsprechenden Implantatlagern mittels Einpressen ausgebildet.

Dies ermöglicht eine besonders einfache Implantation des Implantats, da der Chirurg nur entsprechende Implantatlager z.B. durch Bohren herzustellen hat, um dann die Grundkörper durch Einpressen in die Implantatlager ohne die Verwendung von Fixationselementen wie z.B. Schrauben, Dübeln, Pins oder ähnlichen Formkörpern und ohne weitere Bearbeitung stabil verankern zu können. Die so implantierten Grundkörper sind dann in den Implantatlagern im Preßsitz gehalten. Durch dieses Implantationsverfahren ist die Gefahr einer Verletzung oder Beschädigung von Teilen des Implantats während der Implantation sehr stark reduziert.

Darüber hinaus führt die Verwendung allein von Grundkörpern aus Knochenmaterial menschlichen oder tierischen Ursprungs und eines flexiblen Kollagenbands dazu, daß bei bildgebenden diagnostischen Verfahren durch die Fixation des Implantats entstehende Artefakte praktisch ausgeschlossen sind.

Ein besonderer Vorteil des erfindungsgemäßen Implantats besteht darin, daß dessen Material wegen der Verwendung nur von Materialien biologischen Ursprungs keinen Fremdkörper darstellt. Dadurch trägt das Implantat zur Fixation und Fusion zwischen Implantat und Implantatlager bei, indem es sich während der Einheilung in körpereigenes Gewebe umwandelt.

Das erfindungsgemäße Implantat wird dabei zwar auch im Laufe der Zeit abgebaut, doch entstehen dabei keine das Implantatlager schädigenden Stoffe, vielmehr wird das Implantat über einen mittelfristigen Zeitraum durch körpereigenes Gewebe ersetzt, ohne eine Lyse im Implantatlager durch Abbauprodukte zu verursachen.

Vorteilhafte Ausgestaltungen und Weiterbildungen der Erfindung sind in der Beschreibung, den Zeichnungen und den Unteransprüchen beschrieben.

Wenigstens einer der Grundkörper weist einen im wesentlichen zylindrischen Abschnitt und daran anschließend am Implantatende einen konischen Abschnitt auf. Diese Form erlaubt zum einen eine sehr einfache Herstellung des Implantatlagers durch Einbringen einer einfachen Bohrung in den Knochen, in den der Grundkörper zu implantieren ist. Zum anderen ermöglicht der konische Abschnitt am Implantatende, dessen Durchmesser dort kleiner als der des zylindrischen Abschnitts ist, ein einfaches Einführen des Grundkörpers in das Implantatlager. Besonders bevorzugt beträgt die Länge des zylindrischen Abschnitts etwa 2/3 und die Länge des konischen Abschnitts etwa 1/3 der Länge des Grundkörpers, wobei unter Länge die Ausdehnung des Grundkörpers in Richtung der Zylinderachse zu verstehen ist. Weiterhin liegt besonders bevorzugt die Größe des Konuswinkels des konischen Abschnitts zwischen etwa 2° und etwa 20°. Durch diese nur relativ schwache Verjüngung wird ein Einführen des Grundkörpers in das Implantatlager ohne die Gefahr von starken Verkantungen und einer damit einhergehenden Beschädigung des Implantatlagers durch das Implantat gewährleistet.

Weiterhin weist wenigstens einer der Grundkörper Umfangsabschnitte mit einer Riffelung auf, die bevorzugt quer zur späteren Implantationsrichtung verläuft. Durch eine solche Riffelung wird ein besonders fester Sitz des Grundkörpers in dem Implantatlager sichergestellt. Besonders bevorzugt weist die Riffelung benachbarte konvexe bzw. linear sägezahnförmige Ausnehmungen und zwischen diesen angeordnete Rippen auf, wobei die Rippen in dem von dem Grundkörper abstehenden Bereich in einer Kante auslaufen. Insbesondere durch die Kantenspitzen, die bei der Implantation durch Einschlagen in das Implantatlager mittels eines Stößels teilweise abgebrochen werden können, dann in den Ausnehmungen der Riffelung verbleiben und sich mit in das Gewebe des Implantatlagers eingraben und verdichten, wird ein besonders sicherer Sitz des Grundkörpers in dem Implantatlager gewährleistet. Dagegen können bei einem etwas zu großen Durchmesser des Implantats und einer flächigen Anlage die Einschlagkräfte so groß sein, daß die Gefahr einer Gewebezerstörung besonders an der Kontaktstelle von Implantationswerkzeug, zum Beispiel Stößel, und Grundkörper besteht. Sollten zwischen dem Gewebe des Implantatlagers und dem Grundkörper im Bereich der Ausnehmungen Hohlräume entstehen, werden diese durch nachwachsendes Gewebe zuerst aufgefüllt, so daß sich zusammen mit den eingegrabenen Kanten auch ein sicherer Sitz aufgrund eines gewissen Formschlusses ergibt. Besonders bevorzugt ist eine Riffelung an beiden Grundkörpern ausgebildet.

Bevorzugt kann das flexible Kollagenband durch Demineralisierung von menschlichem oder tierischem Knochenmaterial gebildet sein, was eine einfachere Variation von Form und/oder Dimension des Bandes erlaubt. Besonders bevorzugt ist das flexible Kollagenband aus menschlichem oder tierischem Sehnenmaterial gebildet, wobei insbesondere das Sehnenmaterial schon mit entsprechendem Knochenmaterial für die Grundkörper verbunden sein kann. Solche Implantate sind besonders einfach herstellbar und weisen darüber hinaus eine besonders gute Festigkeit zwischen Grundkörper und Kollagenband auf.

Das Material der Grundkörper ist bevorzugt im wesentlichen spongiöses Knochenmaterial, das ein besonders gutes Einwachsen erlaubt.

Bevorzugt besteht das erfindungsgemäße Implantat aus prozessiertem, konserviertem und sterilem Knochen- und gegebenenfalls, das heißt soweit das Kollagenband nicht durch Demineralisierung von Knochenmaterial gewonnen ist, Sehnenmaterial humanen Ursprungs, ist also ein sogenannter Allograft. Besonders bevorzugt kann ein erfindungsgemäßes Implantat aber auch aus prozessiertem, konserviertem und sterilem Knochen- und gegebenenfalls Sehnenmaterial tierischen Ursprungs bestehen, und somit ein sogenanntes Xenograft sein. Hierbei eignet sich besonders Knochen- und Sehnenmaterial bovinen, porcinen und equinen Ursprungs, da diese ohne Verletzung des menschlichen Körpers oder Entnahme von Knochen- und Sehnenmaterial aus dem menschlichen Körper gewinnbar sind. Darüber hinaus kann das entsprechende Knochen-Sehnenmaterial auch bei geeigneter Auswahl stabiler sein als entsprechendes menschliches Material.

Bevorzugt wird als Material ein geeignetes allogenes oder xenogenes Knochen- und gegebenenfalls Sehnenmaterial verwendet, das derart prozessiert wird, daß es konserviert, lagerfähig und steril ist, so daß es bestimmungsgemäß ohne die Gefahr von Infektionen eingesetzt werden kann.

Die Konservierung des Knochen- und gegebenenfalls Sehnenmaterials kann beispielsweise mittels Gefriertrocknung erfolgen. Vorzugsweise wird aber das Knochen- und gegebenenfalls Sehnenmaterial durch Lösemitteldehydratisierung vom kollagenen Knochen- und gegebenenfalls Sehnenmaterial mittels eines organischen, mit Wasser mischbaren Lösungsmittels, z.B. Methanol, Ethanol, Propanol, Isopropanol, Aeton, MethylEthylketon oder Gemischen dieser Lösungsmittel erzeugt. Ein solches Verfahren zur Konservierung und Sterilisation ist in dem Patent DE 29 06 650 beschrieben, dessen Inhalt durch diese Bezugnahme in die Offenbarung der vorliegenden Anmeldung aufgenommen wird. So hergestelltes Material weist im histologischen Bild ein dem natürlichen Knochen bzw. der natürlichen Sehne sehr ähnliche Struktur auf, so daß die gewünschten Eigenschaften des kollagenen Knochen- und gegebenenfalls Sehnenmaterials erhalten bleiben. Dies ist darauf zurückzuführen, daß das Verfahren eine Dehydratisierung und Freilegung bis in den Feinbau der Fibrille des kollagenen Knochen- und gegebenenfalls Sehnenmaterials erlaubt.

Nach der Lösemitteldehydratisierung kann das Knochen- und gegebenenfalls Sehnenmaterial vorzugsweise terminal sterilisiert werden, was insbesondere durch Bestrahlung mit Gamma- bzw. Elektronenstrahlen, aber auch durch Ethylenoxid oder thermische Verfahren geschehen kann.

Alternativ kann das Knochen- und gegebenenfalls Sehnenmaterial aber auch durch aseptische Prozessierung von kollagenem Knochen- und gegebenenfalls Sehnenmaterial ohne terminale Sterilisation erzeugt werden.

Vor der Sterilisation wird das Implantat zugerichtet, insbesondere können die Grundkörper durch Bearbeitung mit CNC-gesteuerten Maschinen aus entsprechenden Knochen-Knochen-, bzw. Knochen-Sehnen-, oder Knochen-Sehnen-Knochen-Verbünden hergestellt werden. Durch die so erzielte präzise Zurichtung wird eine zuverlässige Verankerung des Implantats im Implantatlager gewährleistet, ohne das Implantat oder auch das Implantatlager mechanisch zu beschädigen.

Bevorzugt wird ein erfindungsgemäßes Implantat bei einem Verfahren zum Ersatz von Sehnen durch Implantate verwendet, wobei wenigstens einer der Grundkörper in einer Richtung senkrecht zur Implantierungsrichtung einen zwischen etwa 0,1 mm und etwa 3 mm größeren Durchmesser aufweist als eine entsprechende Implantatlageröffnung, in die der Grundkörper implantiert wird. Besonders bevorzugt ist es hierbei, daß der Durchmesser des Grundkörpers etwa 0,5 mm größer als der der Implantatlageröffnung ist.

Durch verschieden große Abmessungen der Grundkörper und des Sehnenanteils eines erfindungsgemäßen Implantats ist natürlich neben einer Anwendung zum Ersatz des Kreuzbandes generell auch eine Anwendung beim Ersatz anderer Sehnen möglich, wie z.B. der Ersatz der Achillessehne.

Eine bevorzugte Ausführungform der Erfindung wird nun beispielhaft anhand der Zeichnungen beschrieben. Es zeigen:
- Fig. 1: eine schematische perspektivische Ansicht eines Implantats zum Ersatz einer Sehne nach einer bevorzugten Ausführungsform der Erfindung,
- Fig. 2: einen Ausschnitt aus Fig. 1, der dort mit A **gekennzeichnet** ist und schematisch die Riffelung des Grundkörpers zeigt,
- Fig. 3: eine schematische Draufsicht auf den linken Grundkörper in Fig. 1, und
- Fig. 4: eine schematische Seitenansicht des rechten Grundkörpers in Fig. 1.

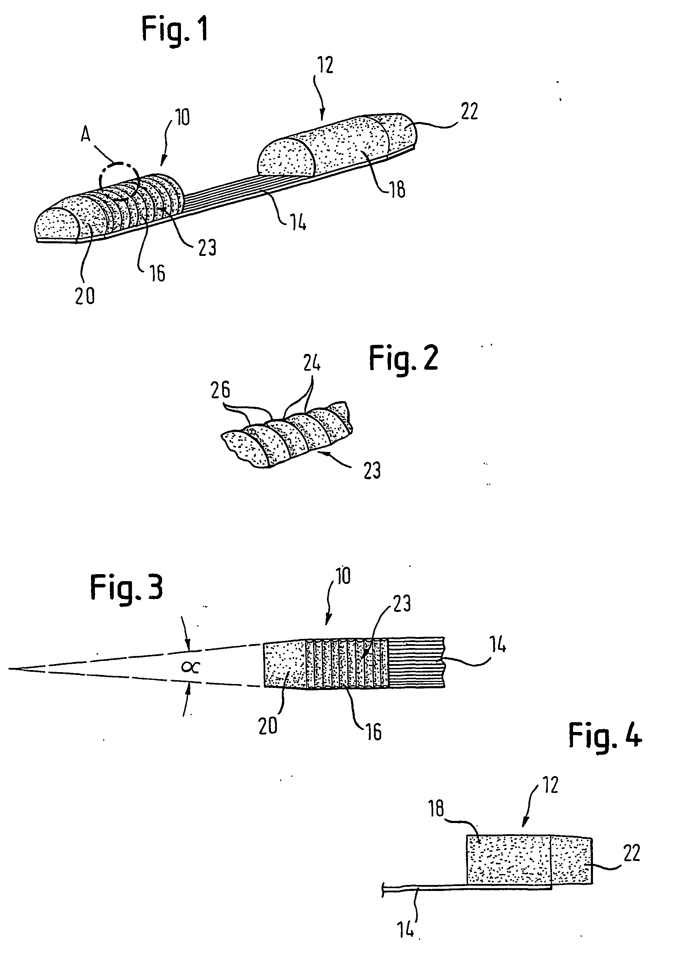

In Fig. 1 weist ein Implantat nach einer bevorzugten Ausführungsform der Erfindung einen ersten Grundkörper 10 und einen zweiten Grundkörper 12 auf, die durch ein flexibles Kollagenband 14 miteinander verbunden sind. Die Grundkörper 10 und 12 bestehen je aus kortiko-spongiösem Knochenmaterial porcinem Ursprungs. Das flexible Kollagenband 14 besteht aus einem Sehnenmaterial allogenen oder xenogenen Ursprungs.

Wie in den Fig. 1, 3 und 4 dargestellt, sind die Knochenblöcke 10 und 12 auf ca. 2/3 ihrer Länge im wesentlichen zylindrisch und jeweils zu den Implantatenden hin auf ca. 1/3 ihrer Länge konisch geformt. Der Konuswinkel α, der nur in Fig. 3 dargestellt aber für beide Grundkörper ungefähr gleich groß ist, beträgt etwa 10°.

Das flexible Kollagenband 14 ist, wie in den Fig. 1 und 4 gezeigt, über die gesamte Länge der entsprechenden zylindrischen Abschnitte 16 und 18 mit den jeweiligen Grundkörpern 10 bzw. 12 verbunden.

Der zylindrische Abschnitt 16 des Grundkörpers 10 weist, wie in den Fig. 1 und 2 gezeigt, eine senkrecht zur Zylinderachse verlaufende Riffelung 23 auf, bei der benachbarte konvexe Ausnehmungen 24 durch Rippen 26 getrennt sind. Das Profil der Rippen läuft an deren, vom Grundkörper 10 weg weisenden Ende spitz unter Ausbildung einer Kante zu. Hierdurch wird ein besonders sicherer Sitz des Implantats im Implantatlager gewährleistet.

Grundsätzlich sind die Außendurchmesser des Implantats, d.h. insbesondere die Durchmesser der zylindrischen Abschnitte 16 und 18 an die Dimension des Implantatlagers angepaßt. Mögliche Dimensionen des beschriebenen Implantats können je nach Einsatzort z.B. die folgenden sein:

Die Gesamtlänge kann zwischen 75 und 200 mm variieren, wobei die Länge der freien Sehne zwischen 20 und 100 mm und deren Breite zwischen etwa 4 und 30 mm liegt. Der Durchmesser der Grundkörper ist nicht geringer als die Breite der Sehne und kann daher auch im Bereich zwischen 34 mm liegen. Die Länge der Grundkörper kann dabei zwischen 15 und 40 mm liegen.

### Bezugszeichenliste

- 10: erster Grundkörper
- 12: zweiter Grundkörper
- 14: flexibles Kollagenband
- 16: zylindrischer Abschnitt
- 18: zylindrischer Abschnitt
- 20: konischer Abschnitt
- 22: konischer Abschnitt
- 23: Riffelung
- 24: Ausnehmung
- 26: Rippe

- α: Konuswinkel

## Patentansprüche

1. Implantat zum Ersatz von Sehnen, insbesondere des vorderen Kreuzbandes, mit einem ersten und einem zweiten Grundkörper (10, 12) aus jeweils spongiösem und/oder kortikalen und/oder kortiko-spongiösem Knochenmaterial menschlichen oder tierischen Ursprungs, die über ein flexibles Kollagenband (14) verbunden und zur Fixation in einem entsprechenden Implantatlager ausgebildet sind,
**dadurch gekennzeichnet,**
**daß** wenigstens einer der Grundkörper (10, 12) einen im wesentlichen zylindrischen Abschnitt (16, 18) und daran anschließend am Implantatende einen konischen Abschnitt (20, 22) aufweist, und
**daß** dieser Grundkörper (10, 12) Umfangsabschnitte mit einer Riffelung (23) aufweist, um eine Verankerung des Grundkörpers in einem Implantatlager mittels Preßsitz zu ermöglichen.

2. Implantat nach Anspruch 1,
**dadurch gekennzeichnet,**
**daß** die Länge des zylindrischen Abschnitts (16, 18) etwa 2/3 und die Länge des konischen Abschnitts etwa 1/3 der Länge des Grundkörpers aufweisen.

3. Implantat nach einem der Ansprüche 1 oder 2,
**dadurch gekennzeichnet,**
**daß** die Größe des Konuswinkels (α) des konischen Abschnitts (20, 22) zwischen etwa 2° und etwa 20° liegt.

4. Implantat nach Anspruch 1,
**dadurch gekennzeichnet,**
**daß** die Riffelung (23) benachbarte konvexe Ausnehmungen (24) und zwischen diesen angeordnete Rippen (26) aufweist, wobei die Rippen in dem von dem Grundkörper abstehenden Bereich in einer Kante auslaufen.

5. Implantat nach einer der vorhergehenden Ansprüche,
**dadurch gekennzeichnet,**
**daß** das flexible Kollagenband (14) durch Demineralisierung von menschlichem oder tierischem Knochenmaterial gebildet ist.

6. Implantat nach einem der Ansprüche 1 bis 4,
**dadurch gekennzeichnet,**
**daß** das flexible Kollagenband (14) aus menschlichem oder tierischem Sehnenmaterial gebildet ist.

7. Implantat nach einem der vorhergehenden Ansprüche,
**dadurch gekennzeichnet,**
**daß** das Material der Grundkörper (10, 12) spongiöses Knochenmaterial ist.

8. Implantat nach einem der vorhergehenden Ansprüche,
**dadurch gekennzeichnet,**
**daß** es durch Bearbeitung mit CNC-gesteuerten Maschinen erhalten ist.

## Claims

1. An implant for the replacement of tendons, in particular of the anterior cruciate ligament, having a first and a second base body (10, 12) made respectively of spongious and/or of cortical and/or of cortico-spongious bone material of human or animal origin which are connected via a flexible collagen ligament (14) and are designed for fixation into a corresponding implant bed,
**characterized**
**in that** at least one of the base bodies (10, 12) has a substantially cylindrical section (16, 18) and a conical section (20, 22) adjoining it at the implant end; and
**in that** said base body (10, 12) has peripheral sections with a ribbing (23) in order to permit an anchorage of the base body in an implant bed by means of a press fit.

2. An implant in accordance with claim 1, **characterized in that** the length of the cylindrical section (16, 18) has approximately 2/3 of the length of the base body and the length of the conical section has approximately 1/3 of the length of the base body.

3. An implant in accordance with one of the claims 1 or 2, **characterized in that** the magnitude of the cone angle (α) of the conical section (20, 22) lies between approximately 2° and approximately 20°.

4. An implant in accordance with claim 1, **characterized in that** the corrugation (23) has adjacent convex recesses (24) and ribs (26) arranged between them, with the ribs running out in an edge in the region projecting from the base body.

5. An implant in accordance with any one of the preceding claims, **characterized in that** the flexible collagen ligament (14) is made by demineralization of human or animal bone material.

6. An implant in accordance with an< one of the claims 1 to 4, **characterized in that** the flexible collagen ligament (14) is formed from human or animal tendon material.

7. An implant in accordance with any one of the preceding claims, **characterized in that** the material of the base bodies (10, 12) is spongious bone material.

8. An implant in accordance with any one of the preceding claims, **characterized in that** it is obtained by machining with CNC controlled machines.

## Revendications

1. Implant pour le remplacement de ligaments, en particulier le ligament croisé antérieur, comprenant un premier et un second corps de base (10, 12) respectivement en matériau osseux spongieux et/ou cortical et/ou cortico-spongieux d'origine humaine ou animale, qui sont reliés via une bande de collage flexible (14) et sont réalisés pour la fixation dans un logement d'implant correspondant,
**caractérisé en ce que**
l'un au moins des corps de base (10, 12) comprend un tronçon essentiellement cylindrique (16, 18) et à la suite de celui-ci un tronçon conique (20, 22) à l'extrémité de l'implant, et **en ce que** ce corps de base (10, 12) comporte des tronçons de périphérie pourvus de stries (23), pour permettre un ancrage du corps de base dans un logement d'implant par engagement à la presse.

2. Implant selon la revendication 1,
**caractérisé en ce que** la longueur du tronçon cylindrique (16, 18) est environ 2/3 de la longueur du corps de base, et la longueur du tronçon conique est environ 1/3 de la longueur du corps de base.

3. Implant selon l'une des revendications 1 ou 2,
**caractérisé en ce que** la taille de l'angle conique (α) du tronçon conique (20, 22) est entre environ 2° et environ 20°.

4. Implant selon la revendication 1,
**caractérisé en ce que** les stries (23) comprennent des évidements convexes voisins (24) et des nervures (26) agencées entre ceux-ci, lesdites nervures se terminant par une arête dans la zone qui dépasse du corps de base.

5. Implant selon l'une des revendications précédentes,
**caractérisé en ce que** la bande de collage flexible (14) est formée par déminéralisation de matériau osseux humain ou animal.

6. Implant selon l'une des revendications 1 à 4,
**caractérisé en ce que** la bande de collage flexible (14) est formée à partir de matériau ligamentaire humain ou animal.

7. Implant selon l'une des revendications précédentes,
**caractérisé en ce que** le matériau du corps de base (10, 12) est du matériau osseux spongieux.

8. Implant selon l'une des revendications précédentes,
**caractérisé en ce qu'**il est obtenu par usinage avec des machines à commande numérique.
